(19) Europäisches Patentamt
European Patent Office
Office européen des brevets

(11) **EP 4 360 548 A1**

(12) **EUROPEAN PATENT APPLICATION**

(43) Date of publication:
**01.05.2024 Bulletin 2024/18**

(21) Application number: **22204263.2**

(22) Date of filing: **27.10.2022**

(51) International Patent Classification (IPC):
**A61B 5/08** *(2006.01)*    **A61B 5/083** *(2006.01)*
**A61B 5/00** *(2006.01)*

(52) Cooperative Patent Classification (CPC):
**A61B 5/0803; A61B 5/082; A61B 5/0823;**
**A61B 5/083; A61B 5/7264; A61B 5/742;**
A61B 2562/0204

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **New Senses Uzay Teknoloji Ve**
**Saglik Ararstirmalari A.S.**
**Istanbul (TR)**

(72) Inventor: **The designation of the inventor has not yet been filed**

(74) Representative: **Demirkiran, Hasan**
**Kordinat Inovasyon ve Fikri Mulkiyet Yonetimi**
**Ltd.**
**Sti.**
**Nispetiye Mah. Nispetiye Cad. No:6 K:2 Etiler**
**Besiktas**
**34340 Istanbul (TR)**

(54) **DIAGNOSIS OF SOME DISEASES WITH SOUND FREQUENCIES**

(57) The invention is a device (1) for the diagnosis of pulmonary diseases and viral infections, vocal cord related diseases, neurological diseases, and laryngeal cancer, mesopharyngeal cancer from cough, phonation, and breath sound, characterized by a central control unit (1.1), a microphone (2) for the input of cough sounds, a moisture chamber (3) where the breath condenses before the pH meter to protect the circuit, a reaction chamber (1.2) for the detection of volatile molecules, and a programmable digital processor (4) that makes sense of the data and converts it into digital data.

**Figure 1**

EP 4 360 548 A1

## Description

## Technical Field

The invention relates to a device for detecting diseases using sound frequencies.

The device enables the determination of the sound frequencies of organs and cells within the body as a whole, which cause noise problems in the field of NVH (Noise, Vibration, Harshness).

## State of the Art

Although our body suspends the vibrations of the organs within it, each organ and even each cell emits vibrations at certain intervals and scales. The vibration emission of our sick body elements varies and becomes irregular. There are devices such as stethoscopes that capture sounds. However, the sounds received from these devices are specific to a certain region. For example, although all our organs vibrate, only the heartbeat can be listened to. Although there are many organs in the same region, not all of them can be listened to, and in the lower abdominal region, the sounds can be mixed together. Body listening can include a crowded frequency spectrum resulting from many components. In order to diagnose and solve the problem, it is necessary to determine the frequencies of the higher than the average amplitude sounds (e.g., local peaks) in this spectrum. This requires a great deal of experience and knowledge to determine which health problem can occur in which frequency range. The frequency of the unknown/searched-for problem can be queried in the remaining high amplitude regions or points after the known sounds (peaks) on the spectrum have been separated.

In another state of the art, in the application numbered TR2019/16368 "Kit for the detection of large six Enterohemorrhagic Escherichia coli serotypes by RT-PCR", the invention is related to PCR diagnostic kits and is related to the diagnosis of diseases by taking swabs or blood.

In another state of the art, the invention with the application number TR2019/01360 "Electronic Nose System for Early Diagnosis of Hereditary Metabolic Diseases and Detection of Attacks" relates to a low-cost electronic nose system that provides early diagnosis and detection of attacks of hereditary metabolic diseases by measuring the odor by transmitting the air drawn from the aerochamber containing a mask and spray chamber to the sample container.

## Object and Summary of the Invention

The purpose of this invention is to create a device that enables real-time diagnosis of diseases with the vibrations emitted by our organs.

The purpose of the invention is to provide a method for quickly determining which of several suspected frequencies in a living organism affected by a disease is the desired frequency and which organ is diseased.

One purpose of the invention is to be used while recording sound in an environment containing a noise problem, and the sound is played externally at the frequencies where suspicious peaks occur in the frequency spectrum in real time. Thus, with the amplitude increase to be created in the relevant peak, it can be easily and quickly determined which of the suspicious frequencies is the frequency of the problem sought. If the frequency of the noise problem can be determined, it can be known whether the relevant organ is working properly. For example, if someone's lung is emitting a disturbing vibration and this vibration can be identified as occurring at a certain frequency (e.g., at 18 Hz), it can be said to be caused by a form of cancer.

It was developed by converting the phonation, cough and breath sounds of the people using the technique into spectrogram graphics in png format by extracting MFCC (Mel Frequency Cepstral Coefficients). More than 70 diseases are classified with this method. The system beneficiary has to perform two validations within the application.

These are;

- Ambient Noise Calibration: If the ambient noise is in the range of 0-10 db, conditions are suitable for testing. Measurement can be performed with high validation. If the ambient noise is above 60 db, the environment is not suitable for testing.
- Anatomical Breath Calibration: The respiratory system is specialized to create gas exchange between blood and atmospheric air.

## Brief Description of the Figures

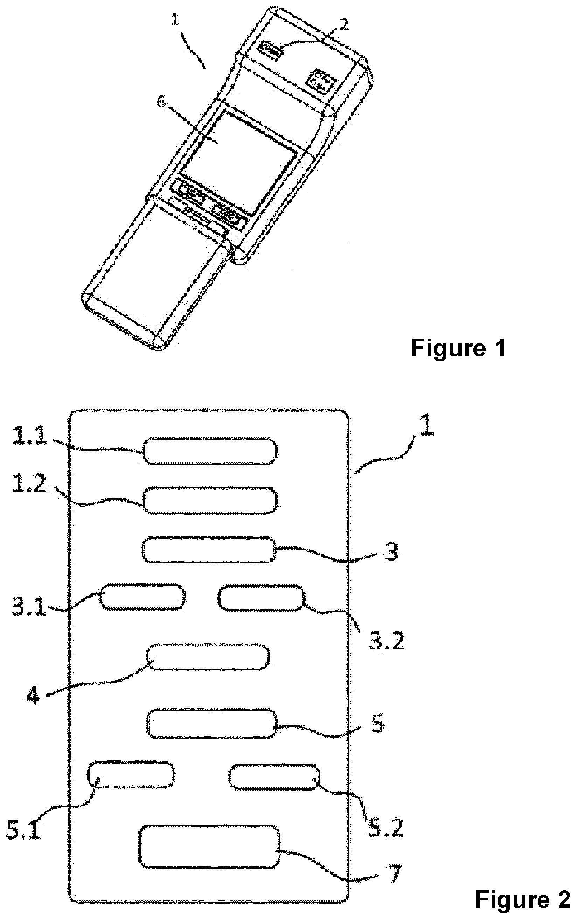

Figure 1    is a representative view of the inventive device.

Figure 2    is a schematic view of the inventive system.

**Reference Numbers**

1-      Device
1.1-    Central Control Unit
1.2-    Reaction chamber
2-      Microphone
3-      Moisture Chamber
3.1-    PH Sensor
3.2-    Moisture Sensor
4-      Processor
5 -     Adapter Chamber
5.1-    Wifi Module
5.2-    Bluetooth Module
6-      Screen
7-      Battery

**Detailed Description of the Invention**

The device (1) has the ability to calculate and diagnose lung-related disorders and viral infections, vocal cord-related diseases, neurological diseases and laryngeal cancer, mesopharyngeal cancer from cough, phonation and breath sound and to calculate reference values related to our immunity by the system with continuous learning software. In its most general form, the device consists of a central control unit (1.1) that modulates the data, a microphone (2) that provides the input of voice data, a moisture chamber (3) where the breath condenses before the pH meter to protect the circuit, a reaction chamber (1.2) to detect volatile molecules, and a programmable digital processor (4) that makes sense of the data and converts it into digital data.

The said moisture chamber (3) contains a pH sensor (3.1) that contacts the alveolar air and a moisture sensor (3.2) that detects the moisture in the system, respectively. In addition, the adapter chamber (5), where the sensor connections are realized in smd scale, contains the screen (6) where the desired data is displayed and the bluetooth module (5.2) in the adapter chamber (5) that shares the data with the desired device. On the other hand, it contains a wireless module (5.1) that shares the data with the cloud server, a battery (7) that provides energy to the system, and a central control unit (1.1) that uses sound frequencies in the 62-72 MHz range, which enables real-time diagnosis of diseases with the vibrations emitted by our organs.

The central control unit (1.1) is the unit where the main board and system components are configured. Moisture chamber (3) is the chamber that weighs the air used to capture volatile molecules. Ph sensor (3.1) is an artificial intelligence-supported sensor that enables accurate Ph calculation. The moisture sensor (3.2) detects the moisture in the system. On the other hand, a reaction chamber (1.2) has been created to detect volatile molecules. The adapter chamber (5) acts as a kind of fuse chamber where the sensor connections are realized in smd scale.

Working principle of the device (1); a system for voice-based lung cancer diagnosis has been developed using artificial intelligence. Audio frequencies and each sound converted into a picture were analyzed using a Convolutional Neural Network (CNN), one of the algorithms in feature extraction and deep learning, one of the sub-branches of artificial intelligence. The success obtained for the selection of this algorithm shows that the system works with high success.

With the gas exchange in the respiratory system, $CO_2$ formed as a result of metabolism in the cells is given to the atmospheric air, while $O_2$ in the atmospheric air is taken into the blood. Due to the anatomical dead space, gas exchange occurs only in 350 ml of the 500 ml of air taken into the lungs with each breath. As the air passes through the larynx, sounds are produced by the vibration of the vocal cords in the larynx. As can be understood during speech, 350 ml of $CO_2$ and 150 ml of $O_2$ are returned to the air.

Our study is to estimate $O_2$ by calculating the oxygen level of the air given with artificial intelligence support within the physiology of the respiratory system. In this way, it is also possible to calculate values such as $CO_2$ and pH. Our clinical experience with this software is directly related to the $O_2$ and $CO_2$ exchange between the blood in the systemic capillaries and tissue cells as a result of internal respiration, and the ability of the veins to deliver venous blood loaded with $CO_2$ to the right side of the heart. If the veins function correctly despite the effects of gravity etc., pH increases as $CO_2$ is eliminated. Otherwise, the increase in $CO_2$ decreases the pH value and causes acidosis. Acid-Base balance disorder is an important cardiovascular data. In such a case, serious conditions occur such as confusion, cardio-pulmonary arrest, congestive heart failure. The mathematical data obtained as a result of these processes are converted into reference data for arterial blood gas values with the validating artificial intelligence software embedded in our server. The basis of the mathematical formula for these values is the Henderson-Hasselbalch equation and the equation is; [(LDH) X (pCO$_2$)] / [(HCO$_3$) X (pO$_2$)] << (Hgb).

With device (1), a system for voice-based diagnosis of lung-related disorders and viral infections, vocal cord-related diseases, neurological diseases and laryngeal cancer, mesopharyngeal cancer using artificial intelligence is presented. The picture converted (the Mel frequency Cepstral coefficients of *cough, breath and phonation sounds were extracted and converted into png format spectrogram graphics. The sounds converted to image format were classified with a convolutional neural network, one of the deep learning algorithms in artificial intelligence.*) Each sound was analyzed with a convolutional neural network, one of the algorithms in feature extraction and deep learning, one of the sub-branches of artificial intelligence. Convolutional neural networks are a sub-branch of deep learning and are often used to analyze visual information. Common applications include image and video recognition, image classification in recommender systems, medical image analysis and natural language processing. The results obtained for the selection of this algorithm show that the system works with a high level of success. The calculation system, which can calculate reference values related to your immunity by the system with continuously learning software, works as follows: The invention first converts phonation, cough and breath data into mathematical data. This is done during the transformation of the acquired data into Mel frequency Cepstral coefficients.

Framing: the speech cue is constantly changing as the organs of sound production are displaced in relation to the words. The speech signal should be divided into small segments, called frames, where the parameters are assumed to remain constant. This is because if the FFT is computed over the whole signal, the spectral information of different phonemes is lost. Instead of taking the FFT of the whole signal, the FFT of the frame is calculated. The frame length varies between 10-30 msec. In this range, speech shows fairly constant acoustic characteristics (Karpov, 2003). Overlap is applied to each frame. The overlap rate of the frames is taken between 30% and 75% of the frame length (Kinnunen, 2003). Applying overlap ensures that the sign at the end of the frame does not lose its significance. After averaging out the speech sample, the parts that can be considered constant against speech variations are expressed as follows. The speech signal is divided into speech segments of length N samples. The first frame consists of N samples, while the next frame starts with M samples after the first frame, so that N-M samples overlap (Rabiner and Juang, 1993).

Windowing: The second operation to obtain the Mel frequency Cepstrum coefficients is windowing. The purpose of windowing is to reduce the spectral effects of the framing process. Windowing prevents discontinuities in the frames (Rabiner and Juang, 1993). In this way, the center regions of the sound are strengthened while the edges are weakened. The mathematical expressions of the commonly used Hamming, Hanning, Blackman, Gaussian, rectangular and triangular windowing functions are as follows.

$$w[k+1] = 0.54 - 0.46\cos\left(2\pi\frac{k}{N-1}\right) \qquad k = 0,\ldots\ldots\ldots,N-1$$

**Hanning:**

$$w[k+1] = 0.5\left(1 - \cos\left(2\pi\frac{k}{N-1}\right)\right), \qquad k = 0,\ldots\ldots\ldots,N-1$$

**Blackman:**

$$w[k+1] = 0.42 - 0.5\cos\left(2\pi\frac{k}{N-1}\right) + 0.08\cos\left(4\pi\frac{k}{N-1}\right), \quad k = 0,\ldots\ldots\ldots,N-1$$

**Gauss:**

$$w[k+1] = e^{-\frac{1}{2}\left(\alpha\frac{k-\frac{N}{2}}{N/2}\right)^2} \quad 0 \leq k \leq N \qquad \text{and} \quad \alpha \geq 2$$

**Rectangular:**

$$w[k+1] = 1, \qquad k = 0,\ldots\ldots,N-1$$

**Triangular:**

N for odd;

$$w[k] = \begin{cases} \dfrac{2k}{N+1}, \ldots\ldots\ldots 1 \leq k \leq \dfrac{N+1}{2} \\ \dfrac{2(N-k+1)}{n+1}, \ldots \dfrac{N+1}{2} \leq k \leq N \end{cases}$$

N for even;

$$w[k] = \begin{cases} \dfrac{2k-1}{N}, \ldots\ldots\ldots 1 \leq k \leq \dfrac{N}{2} \\ \dfrac{2(N-k+1)}{N}, \ldots\ldots \dfrac{N}{2}+1 \leq k \leq N \end{cases} \quad k = 0,\ldots\ldots,N-1$$

## Claims

1. A device (1) for the diagnosis of pulmonary diseases and viral infections, vocal cord related diseases, neurological diseases and laryngeal cancer, mesopharyngeal cancer from cough, phonation and breath sound, wherein the device (1) comprises;

   - central control unit (1.1) that modulates the data,
   - microphone (2) for the input of cough sounds,
   - moisture chamber (3) where breath condenses before the pH meter to protect the circuit,
   - reaction chamber (1.2) to enable the detection of volatile molecules,
   - programmable digital processor (4) that makes sense of the data and converts it into numerical data.

2. The device (1) according to claim 1, **characterized in that** said humidity chamber (3) respectively comprises a pH sensor (3.1) with which the alveolar air is in contact.

3. The device (1) according to claim 1, **characterized in that** it comprises a moisture sensor (3.2) that detects the moisture in the system.

4. The device (1) according to claim 1, **characterized in that** it comprises an adapter chamber (5) in which the sensor connections are realized in smd scale.

5. The device (1) according to claim 1, **characterized in that** it comprises a screen (6) on which the desired data is displayed.

6. The device (1) according to claim 1, **characterized in that** it comprises a bluetooth module (5.2) in the adapter chamber (5) that shares data with the desired device.

7. The device (1) according to claim 1, **characterized in that** it comprises a wireless module (5.1) that shares data with the cloud server.

8. The device (1) according to claim 1, **characterized in that** it comprises a battery (7) that provides energy to the system.

9. The device (1) according to claim 1, **characterized in that** it comprises a central control unit (1.1) that uses sound frequencies in the range of 62-72 MHz, which enables real-time diagnosis of diseases with the vibrations emitted by our organs.

10. The device (1) according to claim 1, **characterized in that** it comprises an artificial intelligence that analyzes a convolutional neural network from algorithms that transform each cough sound into a picture.

**Figure 1**

**Figure 2**

Europäisches
Patentamt

European
Patent Office

Office européen
des brevets

# EUROPEAN SEARCH REPORT

Application Number

EP 22 20 4263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/064449 A2 (RESMED SENSOR TECH LTD [IE]) 31 March 2022 (2022-03-31)<br>* paragraphs [0029], [0032], [0045], [0058], [0077], [0080], [0127]; figure 1 * | 1-10 | INV.<br>A61B5/08<br>A61B5/083<br>A61B5/00 |
| X | US 2021/345939 A1 (JUMBE NELSON L [US] ET AL) 11 November 2021 (2021-11-11)<br>* paragraphs [0007], [0011], [0249], [0290] * | 1-10 | |
| A | KR 2022 0137436 A (KNU INDUSTRY COOPERATION FOUND [KR])<br>12 October 2022 (2022-10-12)<br>* paragraphs [0007], [0014] * | 1-10 | |

TECHNICAL FIELDS
SEARCHED       (IPC)

A61B

The present search report has been drawn up for all claims

| Place of search | Date of completion of the search | Examiner |
|---|---|---|
| The Hague | 23 March 2023 | Chau, Thoi Dai |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

**EP 22 20 4263**

This annex lists the patent family members relating to the patent documents cited in the above-mentioned European search report.
The members are as contained in the European Patent Office EDP file on
The European Patent Office is in no way liable for these particulars which are merely given for the purpose of information.

**23-03-2023**

| Patent document cited in search report | | Publication date | Patent family member(s) | | Publication date |
|---|---|---|---|---|---|
| WO 2022064449 | A2 | 31-03-2022 | NONE | | |
| US 2021345939 | A1 | 11-11-2021 | CA | 3178244 A1 | 11-11-2021 |
| | | | EP | 4146071 A1 | 15-03-2023 |
| | | | US | 2021345939 A1 | 11-11-2021 |
| | | | US | 2022103922 A1 | 31-03-2022 |
| | | | WO | 2021224888 A1 | 11-11-2021 |
| KR 20220137436 | A | 12-10-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TR 201916368

- TR 201901360